# DEMANDE DE BREVET EUROPEEN

(11) **EP 1 541 119 A2**
(43) Date de publication de la demande: **15.06.2005**
(21) Numéro de dépôt: 05290546.0
(22) Date de dépôt: 10.11.2000
(51) Int. Cl.: A61K 7/11, A61K 7/06

(54) **Composition cosmétique haute tenue comprenant au moins un polymère filmogène**

(30) Priorité: 19.11.1999 FR 9914588
(62) Demande divisionnaire de: 00403136.5
(71) Demandeur: L'OREAL, 75008 Paris (FR)
(72) Inventeur: Rollat-Corvol, Isabelle, 75017 Paris (FR); Samain, Henri, 91570 Bievres (FR)
(74) Mandataire: Bourdeau, Françoise

(57) **Abrégé**

L'invention a pour objet une composition cosmétique comprenant au moins un polymère filmogène ou un mélange de polymères filmogènes dans un milieu cosmétiquement acceptable, ledit polymère filmogène ou ledit mélange de polymères filmogènes présentant, à la concentration des polymères de la composition, un profil mécanique, dans un milieu hydroalcoolique à 20 % en volume en éthanol, défini par au moins :
(i) un angle d'accroche α supérieur à 47°
(ii) si α est supérieur ou égal à 90 °, une force maximale de décollement Fₘₐₓ < 5 Newton.

## Description

L'invention a pour objet une composition cosmétique comprenant au moins un polymère filmogène, celui-ci présentant un profil mécanique particulier. Elle vise également un procédé cosmétique comprenant l'application de cette composition ainsi que l'utilisation de cette composition pour fixer et/ou maintenir la forme de la coiffure.

Les compositions conformes à l'inventions peuvent être appliquées sur la peau, les ongles, les lèvres, et sur les fibres kératiniques, comme les cheveux, les sourcils et les cils.

Au sens de la présente invention, on entend par « compositions de coiffage », des compositions destinées à fixer et/ou à maintenir la forme de la coiffure.

On connaît déjà des compositions cosmétique, comme des compositions de coiffage qui contiennent des polymères solubilisés ou en dispersion dans un solvant, éventuellement en présence d'additifs. Elles sont appliquées sur les cheveux au moyen d'un spray ou à la main. Après évaporation du solvant, en général de l'eau ou de l'alcool, il reste sur les cheveux un matériau, qui produit l'effet de coiffage.

Habituellement, les matières premières constitutives de la composition de coiffage sont choisies de telle sorte que le matériau, résultant du séchage de la composition sur les cheveux, soit solide, sinon rigide et au moins mou.

Lorsque les cheveux sèchent sans sollicitation mécanique, par exemple de façon naturelle, il se forme des soudures entre les cheveux. De ce fait, il est possible de maintenir la coiffure dans une forme souhaitée. On ressent la présence des soudures, servant de points de jonction, en passant la main dans les cheveux.

Si la personne se peigne ou démêle ses cheveux, les soudures se cassent et la forme de la coiffure n'est plus maintenue. Il en est de même si les cheveux sont soumis à des sollicitations mécaniques extérieures, par exemple si la personne fait elle-même des mouvements trop brusques ou si elle subit l'effet d'un coup de vent.

Si après avoir appliqué un produit coiffant, on sèche les cheveux tout en les sollicitant mécaniquement, par exemple en effectuant un brushing, il se forme, non pas des soudures entre les cheveux, mais un revêtement continu qui enveloppe les cheveux. Dans ce cas, l'effet coiffant, plus modéré, disparaît encore plus vite au premier coup de vent ou à la première contrainte subie.

Un autre inconvénient des produits cosmétique, notamment capillaire, de l'état de la technique réside dans le fait que ceux-ci peuvent parfois altérer les fibres kératiniques, comme les cheveux, en les rendant secs et cassants, et en leur faisant perdre leur aspect naturel.

Il existe donc un besoin de trouver des compositions cosmétiques qui fixent les fibres kératiniques, notamment les cheveux, plus durablement que les compositions de l'art antérieur et qui leur apportent des propriétés cosmétiques satisfaisantes.

De manière surprenante et inattendue, la Demanderesse a découvert qu'il est possible de fournir des compositions cosmétiques répondant aux exigences exprimées ci-dessus, en sélectionnant judicieusement les polymères mis en oeuvre pour réaliser la composition cosmétique.

L'invention a pour objet une composition cosmétique comprenant au moins un polymère filmogène ou un mélange de polymères filmogènes dans un milieu cosmétiquement acceptable, ledit polymère filmogène ou ledit mélange de polymères filmogènes présentant, à la concentration des polymères de la composition, un profil mécanique, dans un milieu hydroalcoolique à 20 % en volume en éthanol, défini par au moins :
(i) un angle d'accroche α supérieur à 47°
(ii) si α est supérieur ou égal à 90 °, une force maximale de décollement Fₘₐₓ < 5 Newton.

De préférence, pour ii), Fₘₐₓ < 2 Newton.

Un autre objet de la présente invention concerne un procédé cosmétique comprenant la mise en oeuvre de cette composition.

Encore un autre objet de la présente invention concerne l'utilisation d'une composition cosmétique comprenant au moins un polymère filmogène ou un mélange de polymères filmogènes dans un milieu cosmétiquement acceptable, ledit polymère filmogène ou ledit mélange de polymères filmogènes présentant, à la concentration des polymères de la composition, un profil mécanique, dans un milieu hydroalcoolique à 20 % en volume en éthanol, défini par au moins :
(i) un angle d'accroche α supérieur à 47°
(ii) si α est supérieur ou égal à 90 °, une force maximale de décollement Fₘₐₓ < 5 Newton, avec de préférence pour (ii), Fₘₐₓ < 2N.

Le polymère filmogène particulièrement visé par la présente invention est le polyuréthanne PA Marin UA 200 commercialisé par Sanyo. La présente invention vise également, tout particulièrement, les associations, dans des concentrations particulières, du polymère à motifs acides polyacryliques, Avalure AC315, commercialisé par Goodrich, soit avec le copolymère vinylcaprolactame / PVP / diméthylaminoéthyl / méthacrylate, commercialisé par I.S.P sous l'appellation Gaffix VC 713, soit avec le copolymère siliconé greffé POLYSILICONE 8, commercialisé sous l'appellation VS80 par 3M.

On entend par « Fmax », la force maximale de traction, mesurée à l'aide d'un extensomètre, nécessaire pour décoller les surfaces de 0.95 cm², respectives de deux supports (A) et (B), rigides, inertes, non-absorbants, placés en regard l'un de l'autre ; lesdites surfaces étant enduites préalablement par ladite composition de concentration en matière sèche c, à raison de 4/c mg/cm², séchées pendant 48 heures à 22°C sous une humidité relative de 50%, puis soumises pendant 20 secondes à une compression de 3 Newtons et enfin soumises pendant 60 secondes à une traction de vitesse 10 mm/minute ; c est la concentration en matière sèche dans la composition (en gramme par gramme de composition).

Avantageusement, on utilise des supports (A) et (B) constitués de polyéthylène, de polypropylène, d'alliage métallique ou de verre.

L'angle d'accroche α est déterminé en suivant le protocole décrit ci-après.

On réalise un premier film de 243 cm² en déposant dans une boîte de Pétrie, une quantité de composition telle qu'il reste dans cette boîte, après séchage, 0,8 g de matière. Pour cela, on dépose la composition dans la boîte de Pétrie, on la laisse sécher pendant 48 heures à la température ambiante, à 50% d'humidité relative. Le film obtenu est appelé F₁.

On réalise un assemblage coaxial formé par deux cylindres, un disque en verre formant une pastille et un film F₂.

On utilise un premier cylindre mesurant 0,6 cm de haut et de 2,2 cm de diamètre. Sur l'une des extrémités de ce cylindre, on dispose une pastille mesurant 1,1 cm de diamètre et 0,3 cm de hauteur.

Sur l'autre extrémité de ce premier cylindre, on dispose un deuxième cylindre mesurant 2 cm de hauteur et de 0,5 cm de diamètre.

La masse totale de l'assemblage est de 13,4 g. Le centre de gravité se trouve sur l'axe transversal, à 0,4 cm à partir de la surface extérieure de la pastille.

On réalise un deuxième film F₂ en déposant, sur la face extérieure de la pastille, une quantité de composition telle que, après séchage, la pastille soit recouverte de 4 mg de matière. Le séchage est réalisé en maintenant l'assemblage, pendant 48 heures à la température ambiante, à un taux d'humidité relative de 50 %.

Le film F₁ est fixé sur un plan horizontal. On pose l'assemblage sur le film F₁, de manière amovible, le film F₂ venant complètement en contact avec le film F₁.

On incline le plan horizontal à la vitesse de 11 degrés par secondes, jusqu'à ce que l'assemblage dérape. L'angle d'accroche αᵢ mesuré est l'angle entre le film F₁ et l'horizontale au moment où la surface du film F₂ commence à se déplacer relativement à la surface du film F₁.

On répète l'expérience quatre fois de suite avec une même pastille et on calcule la moyenne des angles αᵢ pour une pastille. On réitère le protocole avec 4 pastilles différentes, et on calcule la moyenne des angles d'accroche αᵢ mesurés, ce qui donne l'angle d'accroche α.

Conformément à l'invention, l'angle α est, de préférence, supérieur à 50°.

Dans les compositions conformes à l'invention, le ou les polymères filmogène(s) sont, de préférence, présents à des concentrations comprises entre 0,05 et 20 % en poids, plus préférentiellement comprises entre 0,1 et 15 % en poids, et plus préférentiellement encore entre 0,25 et 10 % en poids par rapport au poids total de la composition.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les épaississants, les tensioactifs anioniques, non ioniques, cationiques ou amphotères, les parfums, les conservateurs, les filtres solaires, les protéines, les vitamines, les provitamines, les polymères non fixants anioniques, cationiques, non ioniques ou amphotères, les huiles minérales, végétales ou synthétiques, les cires, les céramides ou pseudocéramides et tout autre additif classiquement utilisé dans les compositions cosmétiques destinées à être appliqué sur les cheveux.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols comprenant un récipient ainsi qu'un moyen de distribution de la composition, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention peuvent être appliquées sur des cheveux secs ou humides.

L'invention pourra être mieux comprise à l'aide des exemples non limitatifs qui suivent et qui constituent des modes de réalisation avantageux des compositions conformes à l'invention.

### EXEMPLE :

On réalise les compositions suivantes conformes à l'invention.

### Composition 1

| | |
|---|---|
| Avalure AC 315 (GOODRICH) | 5 g (matière active) |
| Gaffix VC 713 (I.S.P.) | 2 g (matière active) |
| Eau qs | 100 g |

### Composition 2

| | |
|---|---|
| PA Marin UA-200 ( SANYO) | 5 g (matière active) |
| Eau qs | 100 g |

On mesure l'effet d'accroche et l'effet collant du polymère de la composition 2 ou du mélange de polymères de la composition 1, en évaluant respectivement Fₘₐₓ et α. Les résultats sont rassemblés dans le tableau 1 ci-après.

**Tableau 1**

| Composition | Effet d'accroche α (°) | Effet collant Fₘₐₓ(Newton) |
|---|---|---|
| 1 | 50° | 0,3 |
| 2 | 90° * | 4,2 |

| | | |
|---|---|---|
| * : à 90 ° l'assemblage n'a pas glissé. L'expérience a été stoppée. | | |

Les deux compositions 1 et 2 conformes à l'invention confèrent, après application sur cheveux naturels, un très bon maintien naturel, non collant et de très longue durée.

## Revendications

1. Composition cosmétique comprenant au moins un polymère filmogène ou un mélange de polymères filmogènes dans un milieu cosmétiquement acceptable, ledit polymère filmogène ou ledit mélange de polymères filmogènes présentant, à la concentration des polymères de la composition, un profil mécanique, dans un milieu hydroalcoolique à 20 % en volume en éthanol, défini par au moins :
(i) un angle d'accroche α supérieur à 47°
(ii) si α est supérieur ou égal à 90 °, une force maximale de décollement Fₘₐₓ < 5 Newton.

2. Composition selon la revendication 1, **caractérisée par le fait que**, pour ii), la force maximale de décollement Fₘₐₓ est inférieure à 2N.

3. Composition selon l'une quelconque des revendications précédentes, **caractérisée par le fait que** l'angle α est supérieur à 50°.

4. Composition selon l'une quelconque des revendications précédentes, **caractérisée par le fait que** le ou les polymères filmogène(s) est (sont) présent(s) à des concentrations comprises entre 0,05 et 20 % en poids, plus préférentiellement comprises entre 0,1 et 15 % en poids, et plus préférentiellement encore entre 0,25 et 10 % en poids par rapport au poids total de la composition.

5. Composition selon l'une quelconque des revendications précédentes, **caractérisée par le fait que** le milieu cosmétiquement acceptable est constitué par de l'eau ou un ou plusieurs solvants cosmétiquement acceptables, tels que des alcools ou des mélanges eau-solvant(s), ces solvants étant de préférence des alcools en C₁-C₄.

6. Composition selon l'une quelconque des revendications précédentes, **caractérisée par le fait qu'**elle comprend, en outre, au moins un moins un additif choisi parmi les épaississants, les tensioactifs anioniques, non ioniques, cationiques ou amphotères, les parfums, les conservateurs, les filtres solaires, les protéines, les vitamines, les provitamines, les polymères non fixants anioniques, cationiques, non ioniques ou amphotères, les huiles minérales, végétales ou synthétiques, les cires, les céramides ou pseudocéramides et tout autre additif classiquement utilisé dans les compositions cosmétiques destinées à être appliqué sur les cheveux.

7. Dispositif aérosol comprenant une composition conforme à l'une quelconque des revendications précédentes.

8. Procédé cosmétique, **caractérisé par le fait qu'**il comprend l'application d'une composition selon l'une quelconque des revendications 1 à 6.

9. Utilisation d'une composition selon l'une quelconque des revendications 1 à 6 pour fixer et/ou mettre en forme la coiffure.
